# EUROPEAN PATENT APPLICATION

(11) **EP 3 632 500 A1**
(43) Date of publication of application: **08.04.2020**
(21) Application number: 18805575.0
(22) Date of filing: 21.05.2018
(51) Int. Cl.: A61M 37/00, A61K 8/02, A61K 8/65, A61K 8/73, A61K 8/85, A61Q 19/00

(54) **WATER-SOLUBLE COSMETIC SHEET RETAINING FILM**

(30) Priority: 22.05.2017 JP 2017100644
(71) Applicant: Cosmed Pharmaceutical Co., Ltd., Kyoto-shi, Kyoto 601-8014 (JP)
(72) Inventor: QUAN, Ying-shu, Kyoto-shi Kyoto 601-8014 (JP); TANAKA, Hiroshi, Kyoto-shi Kyoto 601-8014 (JP); KONDOU, Naoko, Kyoto-shi Kyoto 601-8014 (JP); KAMIYAMA, Fumio, Kyoto-shi Kyoto 601-8014 (JP)
(74) Representative: Ter Meer Steinmeister & Partner
(86) International application number: PCT/JP2018/019474
(87) International publication number: WO 2018/216652

(57) **Abstract**

To provide a means capable of applying a water-soluble cosmetic sheet including a microneedle array without using an adhesive sheet.

A repeelable support film for a microneedle patch, having a pattern-coated adhesive face on one side, and coming in contact with a back face of a substrate of a microneedle array through the adhesive face; a repeelable support film for a water-soluble cosmetic sheet, having a pattern-coated adhesive face on one side, and coming in contact with a back face of the cosmetic sheet containing a water-soluble polymer through the adhesive face; a microneedle patch composed of a microneedle array containing a water-soluble polymer or a water-swellable resin as a base, and a support film, wherein the support film has a pattern-coated adhesive face on one side and adheres to a back face of a substrate of the microneedle array through the adhesive face; and a water-soluble sheet-shaped cosmetic composed of a water-soluble cosmetic sheet containing a water-soluble polymer and a support film, wherein the support film has a pattern-coated adhesive face on one side and adheres to a back face of the water-soluble cosmetic sheet through the adhesive face.

## Description

### Technical Field

The present invention relates to a technique of storing a water-soluble cosmetic sheet with no adhesive sheet, or a technique of holding the water-soluble cosmetic sheet during transport.

### Background Art

The water-soluble cosmetic sheet is a thin sheet composed of a water-soluble polymer and may contain various beauty components therein. This product exhibits beauty effects through a process that each one of the sheets is taken out and brought into close contact with a face, and then the face is massaged with a lotion from a back side to dissolve the sheet on the face. Main examples of the water-soluble cosmetic sheet include a sheet made of hyaluronic acid, a sheet made of collagen, a sheet made of alcogel, as well as a microneedle patch. The microneedle patch is a sheet in which microneedles stand closely together on a flexible substrate. The sheet may have various shapes such as circle, oval, comma shape and rectangle and have an area of about 1 cm² to 40 cm². Since these microneedle sheets have microneedles on one side and needle tips are extremely sharp, the tips are extremely weak against mechanical stimulations. Thus, in order to store and transport the microneedle sheet, it is necessary to exercise ingenuity for avoiding damages of the tips. Even in the case of a water-soluble cosmetic sheet having no needles like the microneedle sheet, it is difficult to reliably and safely protect the thin and small sheet without a lot of trouble.

For that reason, the following methods have been taken so far. For example, a protective release sheet has been developed, which has a substrate as a part of a flexible microneedle patch, also serves as a part for holding a microneedle array during pasting on a skin or storage in a container, has one or a plurality of holes inside and one or a plurality of cutting lines from the outer edge toward the holes, and holds a plurality of microneedles of the patch in the holes (Patent Document 1). The protective release sheet is attached to an adhesive face on a back face of the microneedle array, and the adhesive face is pasted on a skin while releasing the protective release sheet so as to bring the microneedle array into close contact with the skin. In addition, a dedicated storage container for storing and transporting microneedle patches held by protective release sheets has been developed (Patent Documents 2 and 3). Also, a storage container capable of holding and containing the adhesive sheet portion of the microneedle patch by a trestle portion has been reported (Patent Document 4). Furthermore, a packaging body for a microneedle sheet has also been developed, in which a plurality of microneedles of the microneedle sheet are formed using a plurality of micro recesses on the sheet member, and then also protected by the plurality of micro recesses (Patent Document 5).

### Prior Art Documents

### Patent Documents

Patent Document 1: Japanese Patent Application Laid-Open No. 2014-028108
Patent Document 2: Japanese Patent Application Laid-Open No. 2012-213586
Patent Document 3: Japanese Patent Application Laid-Open No. 2014-079622
Patent Document 4: Japanese Patent Application Laid-Open No. 2015-061677
Patent Document 5: Japanese Patent Application Laid-Open No. 2016-067618

### Summary of Invention

### Problem to be solved

In all existing methods, a microneedle array was held by protecting a back face of the microneedle array with an adhesive sheet, and bonding the adhesive face of the adhesive sheet to a container while holding the adhesive sheet on a protective release sheet or without using the protective release sheet. However, in that case, when administering the microneedles to a human, an integrated combination of the microneedle array and the adhesive sheet was necessarily administered. In a case of a microneedle array composed of a resin capable of dissolving or swelling in vivo, if only the microneedle array can be easily administered to a human skin without an adhesive sheet, dissolution of the microneedles in the skin is significantly accelerated by supplying water to the microneedle array from the back face, which is advantageous for exerting the effect. An object of the present invention is to provide a means for applying a microneedle array without an adhesive sheet. In cases of sheets other than the microneedle array, it is common that each one of sheets is put into a bag for storage and transport, but there have been drawbacks such as inconvenience in taking out a thin sheet, and inconvenience in applying the sheet to the face. Although the following description refers to a microneedle patch in the widest range of application field according to the present invention, the gist and application of the present invention are not limited to the microneedle patch but covers general water-soluble cosmetic sheets.

### Solution to Problem

In light of the above-mentioned circumstance, the present inventors have continued to intensely study, thereby succeeded in bonding, holding and applying a water-soluble cosmetic sheet including a microneedle array by forming a small-area adhesive face on a support film without using a sheet-shaped adhesive, and completed the present invention.

The present invention is as follows.
[1] A repeelable support film for a microneedle patch, having a pattern-coated adhesive face on one side, and coming in contact with a back face of a substrate of a microneedle array through the adhesive face.
[2] A repeelable support film for a water-soluble cosmetic sheet, having a pattern-coated adhesive face on one side, and coming in contact with a back face of the cosmetic sheet containing a water-soluble polymer through the adhesive face.
[3] The support film according to [1], wherein an area of the pattern-coated adhesive face of the support film accounts for 0.1% to 30% of an area of the microneedle array.
[4] The support film according to [2], wherein an area of the pattern-coated adhesive face of the support film accounts for 0.1% to 30% of an area of the water-soluble cosmetic sheet.
[5] The support film according to any one of [1] to [4], wherein the pattern-coated adhesive face of the support film has a probe tack value of 0.1 N to 3.0 N.
[6] A microneedle patch composed of a microneedle array containing a water-soluble polymer or a water-swellable resin as a base, and a support film, wherein the support film has a pattern-coated adhesive face on one side and adheres to a back face of a substrate of the microneedle array through the adhesive face.
[7] A water-soluble sheet-shaped cosmetic composed of a water-soluble cosmetic sheet containing a water-soluble polymer and a support film, wherein the support film has a pattern-coated adhesive face on one side and adheres to a back face of the water-soluble cosmetic sheet through the adhesive face.
[8] The microneedle patch according to [6], wherein an area of the pattern-coated adhesive face of the support film accounts for 0.1% to 30% of an area of the microneedle array.
[9] The water-soluble sheet-shaped cosmetic according to [7], wherein an area of the pattern-coated adhesive face of the support film accounts for 0.1% to 30% of an area of the water-soluble cosmetic sheet.
[10] The microneedle patch or the water-soluble sheet-shaped cosmetic according to any one of [6] to [9], wherein the pattern-coated adhesive face of the support film has a probe tack value of 0.1 N to 3.0 N.

### Effects of Invention

In the present invention, the microneedle patch is prepared by bonding a support film pattern-coated with an adhesive to a back face of a microneedle array. By the pattern-coating adhesion, characteristically the adhesion area of the microneedle array with the support film is made significantly smaller than the adhesion area with the support film having the entire face coated with an adhesive. Thereby, the adhesive force can be extremely reduced despite of using an adhesive having the same adhesive strength with the microneedle array per unit area on the adhesive surface.

According to the present invention, the support film is easily peeled immediately before applying the microneedle patch to a skin, so that only the microneedle array can be applied to the skin. Alternatively, after applying the microneedle patch with the support film to the skin, the support film can be easily detached from the skin while holding the end portion of the microneedle array applied to the skin with one hand.

In a case of a water-soluble cosmetic sheet other than the microneedle array, the back face of the sheet is held by the adhesive pattern-coated on the support film, so that stable storage and transportation can be achieved. In use, after applying the sheet to a face, the support film is detached from the skin while holding the end portion of the sheet with one hand. Thereafter, the sheet is dissolved on the face by massage with a lotion.

### Brief Description of Drawings

Figure 1 illustrates examples of pattern coating with an adhesive. A: circle shape (dots), B: vertical stripe shape, C: curved shape, D: lattice shape.
Figure 2 illustrates a schematic drawing of a microneedle patch in Example 1.

### Description of Embodiments

### (Support film)

The support film according to the present invention is a support film for a microneedle patch, which has a pattern-coated adhesive face on one side and is used in contact with a back face of a substrate of a microneedle array through the adhesive face. Alternatively, the support film according to the present invention is a support film for a water-soluble cosmetic sheet, which has a pattern-coated adhesive face on one side and is used in contact with a back face of the water-soluble cosmetic sheet through the adhesive face. In the present invention, the microneedle patch is also used as a water-soluble cosmetic sheet.

Examples of materials for the support film include a polyester such as polyethylene terephthalate (PET), a polyolefin such as polyethylene and polypropylene, paper, fabric, non-woven fabric, and the like. The support film either may be or may not be subjected to mold release treatment. The support film has a thickness of 30 to 1,000 µm, preferably 50 to 500 µm. When the thickness is less than 30 µm, handling is inconvenient, and when it is 1,000 µm or more, it may be difficult to peel the film.

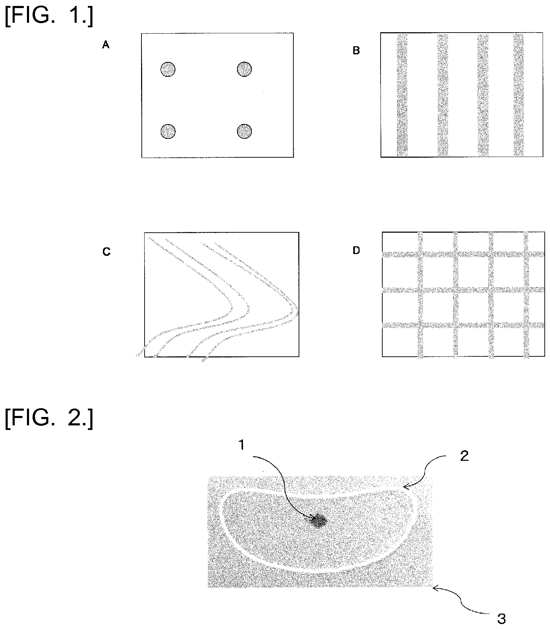

The present invention is characterized in that the area of the adhesive face is reduced by pattern-coating one side of the support film with the adhesive. The pattern of the pattern coating is not particularly limited. Examples of the pattern design include a linear shape, a curved shape, a circular shape, an elliptical shape, an angular shape, and the like. Examples of the pattern are shown in Figure 1. The position of the pattern design on the support film is not particularly limited as long as the pattern design fits within the back face of the microneedle array to be bonded. The coating method of the pattern is not particularly limited. A stencil printing technique such as screen printing, a gravure printing technique, and the like may be used.

An area of the adhesive face formed by pattern coating preferably accounts for 30% or less and 0.1% or more of an area of the microneedle array or the water-soluble cosmetic sheet depending on an adhesive strength of the adhesive to be used. When the area of the adhesive face accounts for 30% or more of the array area, it tends to be difficult to peel the microneedle array or the water-soluble cosmetic sheet from the support film even if the adhesive strength is low. In addition, when the area of the adhesive face accounts for 0.1% or less of the array area, it tends to be unstable to support the microneedle array or the water-soluble cosmetic sheet even if the adhesive strength is high.

The adhesive used in the present invention is not particularly limited. Preferable examples of the adhesive base include a base made of an acrylic adhesive, a base made of a rubber-based adhesive, a base made of a silicone-based adhesive, and the like.

Examples of the acrylic adhesive used for the acrylic adhesive base include a single polymer or copolymer of an alkyl (meth)acrylate obtained from an aliphatic alcohol having 1 to 18, particularly preferably 4 to 18 carbon atoms and a (meth)acrylic acid, and a copolymer of an alkyl (meth)acrylate and another functional monomer.

Examples of the functional monomer include a monomer having a hydroxyl group, a monomer having a carboxyl group, a monomer having an amide group, a monomer having an amino group, and the like. In addition, as a copolymerizable monomer, e.g. vinyl acetate, styrene, α-methyl styrene, vinyl chloride, acrylonitrile, ethylene, propylene, butadiene, and the like can also be used. Preferably, the adhesive contains 50 wt% or more of alkyl (meth)acrylate as a (co)polymerization component.

For the purpose of preparing an acrylic adhesive, solution polymerization of the necessary monomers is usually carried out in the presence of a polymerization initiator. However, the polymerization manner is not limited to the solution polymerization. In addition, the polymerization reaction conditions are appropriately selected mainly depending on the type of the monomer.

The rubber-based adhesive used for the rubber-based adhesive base is exemplified by an adhesive prepared by adding e.g. 20 to 200 parts by weight of a tackifier such as rosin-based resin, polyterpene resin, coumarone-indene resin, petroleum-based resin and terpene-phenol resin, and if necessary, appropriate amounts of a softener such as liquid polybutene, mineral oil, lanolin, liquid polyisoprene and liquid polyacrylate, a filler such as titanium oxide, an antioxidant such as butylhydroxytoluene, and the like, to 100 parts by weight of a rubber elastic body such as natural rubber, styrene-isoprene-styrene block copolymer, styrene-butadiene-styrene block copolymer, styrene-olefin-styrene block copolymer, polyisoprene, polybutene, polyisobutylene and ethylene-vinyl acetate copolymer.

The silicone-based adhesive used for the silicone-based adhesive base is exemplified by an adhesive composed mainly of polydimethylsiloxane and the like.

The support film according to the present invention is used so as to be bonded to the substrate of the microneedle array or the back face of the water-soluble cosmetic sheet through the adhesive face formed of the aforementioned adhesive. It is necessary that the adhesive force between the microneedle array or the water-soluble cosmetic sheet and the support film is high to the extent where they are not peeled off during storage or transportation, and is low to the extent where they are easily peeled during skin application. Since it is complicated to carry out the test of peeling the microneedle array or the water-soluble cosmetic sheet from the support film to which the microneedle array or the water-soluble cosmetic sheet is bonded, the strength of the adhesive force is measured in compliance with Methods of Adhesion Testing (probe tack testing) going to be described in the 17th Revised Japanese Pharmacopoeia. In compliance with this testing method, tackiness of the adhesive face on the support film is measured, which is defined as an adhesive strength. As a result of measuring the probe tack values for various samples, a preferable probe tack value of the adhesive face is 0.1 N to 3.0 N. When a probe tack value for bonding is lower than 0.1 N, there is a risk that the support film is peeled off during transportation or by impact. In addition, when it is higher than 3.0 N, the support film is hard to peel off during use.

### (Microneedle patch)

The microneedle patch according to the present invention is composed of a microneedle array containing a water-soluble polymer or a water-swellable resin as a base, and the support film. The support film has the pattern-coated adhesive face on one side, and is therefore bonded to the back face of the substrate of the microneedle array through the adhesive face.

The water-soluble polymer or water-swellable resin used in the present invention may be any resin which is soluble or swellable in vivo, and examples thereof include a polysaccharide such as glycogen, dextrin, dextran, dextran sulfate, sodium chondroitin sulfate, hydroxypropylcellulose, alginic acid, agarose, chitin, chitosan, pullulan, amylopectin, starch, hyaluronic acid and carboxymethylcellulose, a protein such as collagen, gelatin and albumin, and a synthetic polymer such as polyvinyl alcohol, carboxyvinyl polymer, sodium polyacrylate, polyvinylpyrrolidone and polyethylene glycol, and particularly a polymer substance selected from hyaluronic acid, collagen, starch, gelatin, polyvinylpyrrolidone and polyethylene glycol is preferable. These water-soluble polymers or water-swellable resins may be used alone or in combination of two or more of them.

The hyaluronic acid is a type of glycosaminoglycans (mucopolysaccharides), and has a structure in which disaccharide units of N-acetylglucosamine and glucuronic acid are linked. Examples of the hyaluronic acid include a biological hyaluronic acid isolated from cockscomb, umbilical cord or the like, a cultured hyaluronic acid mass-produced with a lactic acid bacterium, a streptococcus, or the like. In the biological hyaluronic acid, collagen resulting from the original organism cannot be completely removed, and the remaining collagen may have an adverse effect, and therefore a cultured hyaluronic acid containing no collagen is preferable. Thus, a hyaluronic acid preferably contains 50 wt% or more of cultured hyaluronic acid.

When the microneedle array is prepared using, as an ingredient, a water-soluble polymer substance selected from hyaluronic acid, starch, collagen, gelatin, polyvinylpyrrolidone and polyethylene glycol, the microneedle array molded from these polymer substances becomes hard and easily pierces into a skin in a case of a low weight-average molecular weight, but the mechanical strength is reduced and the needles are likely to be broken during storage or piercing into the skin. Conversely, in a case of a high weight-average molecular weight, the mechanical strength is improved and the needles become tougher, and therefore the needles are less likely to be broken during storage or piercing into the skin, but the hardness decreases and the needles are less likely to pierce into the skin. Thus, the weight-average molecular weight is preferably 5,000 to 2,000,000.

For the purpose of preparing a microneedle array which easily pierces into a skin, is less likely to be broken, and easily dissolves in vivo, the microneedle array may be formed from a mixture of a high molecular weight polymer substance having a weight-average molecular weight of 100,000 or more and a low molecular weight polymer substance having a weight-average molecular weight of 50,000 or less. For the weight-average molecular weight of the high molecular weight polymer substance, 100,000 or more is enough, and 2,000,000 or less is preferable. In addition, for the weight-average molecular weight of the low molecular weight polymer substance, 50,000 or less is enough, and 1,000 or more is preferable. In the present invention, the weight average molecular weight is a value measured by gel permeation chromatography (GPC).

Since a ratio of the mixed high molecular weight polymer substance and low molecular weight polymer substance also varies depending on the type and the weight-average molecular weight of each polymer substance, it is only necessary to appropriately determine the ratio so that the mechanical strength and hardness are preferable, but generally it is preferable that a content of the high molecular weight polymer substance is 40 to 95 wt%, and a content of the low molecular weight polymer substance is 60 to 5 wt%.

The microneedle array can be mass-produced using a mold (die). A microneedle array composed of an injection-moldable polymer as a base may be produced by injection-molding a base using a die (e.g. method described in [0017] and [0018] in Japanese Patent Application Laid-Open No. 2003-238347). For the injection molding die, stainless steel, heat resistant steel, superalloy, or the like can be used. A typical die has 100 to 1000 cut portions corresponding to microneedles per square centimeter to form microneedle shapes. For the purpose of forming the cut portions, a fine processing means such as a grinder can be used.

When hyaluronic acid, dextran, polyvinylpyrrolidone, sodium chondroitin sulfate, hydroxypropylcellulose, polyvinyl alcohol, or a mixture thereof is used as a base, a base aqueous solution may be poured into a mold, dried, and then removed (e.g. method described in [0029] to [0031] in Japanese Patent Application Laid-Open No. 2009-273872).

The microneedle array may either contain or be coated with various drugs.

The microneedle array produced as described above can have any shape depending on the application site. Typical examples of the shape include circle, oval, comma shape, and the like. The back face of the substrate of the microneedle array which may have various shapes is integrally bonded to the adhesive face formed on one side of the support film according to the present invention to produce the microneedle patch according to the present invention. Herein, the microneedle array may be equal in size to the support film, or otherwise the support film may be larger than the, or otherwise the support film may be larger than the microneedle array. The shape of the support film is not particularly limited. When the support film is equal in size to the microneedle array, the support film has the same shape as the microneedle array, but when the support film is larger than the microneedle array, the support film may have a shape different from that of the microneedle array.

As described above, in the microneedle patch according to the present invention, the area of the pattern-coated adhesive face on the support film accounts for 0.1% to 30% of the area of the microneedle array.

As described above, in the microneedle patch according to the present invention, the pattern-coated adhesive face on the support film has a probe tack value of 0.1 N to 3.0 N.

The support film is peeled immediately before applying the microneedle patch according to the present invention to a skin, so that only the microneedle array can be applied to the skin. Alternatively, after applying the microneedle patch with the support film to the skin, the support film can be detached from the skin while holding the end portion of the microneedle array applied to the skin with one hand. Although the microneedle patch according to the present invention is free from the adhesive after application to the skin, adherence of the patch with the skin can be kept by skin moisture or appropriate hydration. When the microneedles are intended to be dissolved together with the substrate and absorbed into the skin, the dissolution time is shortened because of the absence of the adhesive layer.

### (Water-soluble sheet-shaped cosmetic)

The water-soluble sheet-shaped cosmetic according to the present invention is composed of a water-soluble cosmetic sheet containing a water-soluble polymer and the support film. Since the support film has a pattern-coated adhesive face on one side, the support film is bonded to the back face of the water-soluble cosmetic sheet through the adhesive face.

The water-soluble polymer constituting the water-soluble cosmetic sheet may be any polymer dissoluble in vivo, and examples of the polymer include hyaluronic acid, collagen, xanthan gum, gellan gum, alginic acid, carboxymethylcellulose, and the like. These water-soluble polymers may be used alone or in combination of two or more of them.

When using the hyaluronic acid as a principal raw material, an aqueous solution containing a hyaluronic acid, a polycarboxylic acid or oxycarboxylic acid, and a polyhydric alcohol is prepared, and the prepared aqueous solution is applied on a film such as polyethylene terephthalate so as to have a uniform thickness, and dried to produce a hyaluronic acid gel sheet having a predetermined thickness. The row material hyaluronic acid preferably has a molecular weight in a range of about 5×10⁴ to 5×10⁶ daltons. As long as the molecular weight is within this range, two or more types of hyaluronic acids having different molecular weights may be mixed for use. Furthermore, the hyaluronic acid having the molecular weight within this range and a hyaluronic acid having a molecular weight smaller than this range can also be mixed for use.

When using the collagen as a as a principal raw material, a collagen aqueous solution is prepared, if necessary, together with a compatibilizer, and the prepared aqueous solution is applied on a film such as polyethylene terephthalate so as to have a uniform thickness, and dried to produce a collagen sheet having a predetermined thickness.

In a case of an alcogel sheet, a carboxy group-containing water-soluble polymer such as xanthan gum, gellan gum, alginic acid, carboxymethylcellulose and hyaluronic acid which have a carboxy group, and a polyhydric alcohol are used. The carboxyl group-containing water-soluble polymer may be partially converted into a sodium salt, a potassium salt, or the like.

The carboxy group-containing water-soluble polymer preferably has a molecular weight in a range of about 5×10⁴ to 5×10⁶ daltons. As long as the molecular weight is within this range, different water-soluble polymers may be mixed, and the same water-soluble polymers having different molecular weights may be mixed for use. Furthermore, the water-soluble polymer having the molecular weight within this range and a water-soluble polymer having a molecular weight smaller than this range can also be mixed for use.

Valuable components such as a cosmetic and a pharmaceutical ingredient can be blended into the water-soluble cosmetic sheet as long as the object and effect of the present invention are not affected. Examples of blendable components include a whitening component, an anti-wrinkle component, an anti-inflammatory component, a blood circulation-promoting component, an antibacterial component, an anti-pruritic component, various vitamins and derivatives thereof, an antioxidant component pigment, an oil component, a perfume, and the like. The cosmetic and pharmaceutical ingredient to be blended may be added to the raw material aqueous solution.

The water-soluble cosmetic sheet produced as described above normally has a thickness of 20 to 1000 µm. The sheet preferably has a thickness of 20 to 200 µm because the sheet is easily dissolved on the skin surface. However, when such a thin film is pasted on the face, the film is difficult to handle. The back face of the cosmetic sheet is supported by a support film, the cosmetic sheet is pasted on the face, and then the support film is peeled. At this time, it is necessary that the cosmetic sheet remains in its original form on the face. The water-soluble cosmetic sheet may have any shape depending on the application site. Typical examples of the shape include circle, oval, comma shape, face shape, and the like. The back face of the water-soluble cosmetic sheet which may have various shapes is integrally bonded to the adhesive face formed on one side of the support film according to the present invention to produce the water-soluble sheet-shaped cosmetic according to the present invention. Herein, the water-soluble cosmetic sheet mat be equal in size to the support film, or otherwise the support film may be larger than the water-soluble cosmetic sheet. The shape of the support film is not particularly limited. When the support film is equal in size to the water-soluble cosmetic sheet, the support film has the same shape as the water-soluble cosmetic sheet, but when the support film is larger than the water-soluble cosmetic sheet, the support film may have a shape different from that of the water-soluble cosmetic sheet.

As described above, in the water-soluble sheet-shaped cosmetic according to the present invention, the area of the pattern-coated adhesive face on the support film accounts for 0.1% to 30% of the area of the water-soluble cosmetic sheet.

As described above, in the water-soluble sheet-shaped cosmetic according to the present invention, the pattern-coated adhesive face on the support film has a probe tack value of 0.1 N to 3.0 N.

The support film is peeled immediately before applying the water-soluble sheet-shaped cosmetic according to the present invention to a skin, so that only the water-soluble cosmetic sheet can be applied to the skin. Alternatively, after applying the water-soluble cosmetic sheet with the support film to the skin, the support film can be detached from the skin while holding the end portion of the water-soluble cosmetic sheet applied to the skin with one hand. After the water-soluble sheet-shaped cosmetic according to the present invention is applied to the skin, adherence of the sheet with the skin can be kept by skin moisture or appropriate moisture supply.

Hereinafter, the present invention will be explained with reference to Examples, however, the present invention is not limited to Examples.

### (Example 1)

A comma-shaped microneedle patch having a long side of 28 mm and a short side of 12 mm was produced by a conventional method. A length of the needles was 200 µm, and an interval of the needles was 600 µm. For the support film for holding the needles, a PET having a thickness of 50 µm was used. For the pattern coating of the adhesive to the support, a PET film having a thickness of 50 µm (manufactured by PANAC Corporation) was used. On the film, another PET film having circular holes with a diameter of 3 mm formed by laser was laid. In relation to the intervals among the holes, the longitudinal interval was 30 mm and the lateral interval was 40 mm. The PET film having holes was brought into close contact with the support film, to which the adhesive was applied so that only the hole portions on the support were coated, to obtain a support having circular adhesive dots with a diameter of 3 mm. For the adhesive, an (acrylate/VP) copolymer (cosmetic grade, manufactured by CosMED Pharmaceutical Co., Ltd.) was used. The support film was cut into a rectangle of 30×40 mm so that an adhesive dot was positioned at the center, to prepare a support film. A comma-shaped microneedle patch was bonded to a support film having the adhesive dot at the center (see Figure 2). The measured probe tack value on the circle adhesive face having the diameter of 3 mm on the support film was 0.9 N. The microneedles bonded to the support film were packaged, and an impact test in which the package was dropped from the top of the desk onto the floor was repeated 10 times. However, no peeling was caused.

In use, the microneedle patch with the support film was pressed against a site around the eye of the face to bring the microneedle patch into close contact with the skin, then the support film was peeled from one side while holding the patch with one hand. At this time, the peeling of the adhesive portion was also smooth.

### (Example 2)

2 g of a low molecular weight collagen (manufactured by Nippi. Inc.) and 8 g of hyaluronic acid were dissolved in 90 g of water, which was casted onto a release PET, and heat-dried to produce a sheet having a thickness of 50 µm. This sheet was cut into an oval having a long side of 40 mm and a short side of 15 mm to produce a sheet-shaped cosmetic. For the support film for holding the sheet-shaped cosmetic, a PET having a thickness of 50 µm was used. The pattern coating on the support with the adhesive was carried out in the same manner as in Example 1, and the support was placed so as to have two adhesive portions with a diameter of 3 mm per a size (50 mm×30 mm). For the support, a PET film having a thickness of 50 µm (manufactured by PANAC Corporation) was used. For the adhesive, an (acrylate/VP) copolymer (cosmetic grade, manufactured by CosMED Pharmaceutical Co., Ltd.) was used. This sheet-shaped cosmetic was pressed and bonded to a support film at two points to prepare a product. In use, the sheet-shaped cosmetic with the support film was pressed against a site around the eye of the face to bring the film-shaped cosmetic into close contact with the skin, then the support film was peeled from one side while holding the sheet with one hand. At this time, the peeling of the adhesive portion was also smooth.

### [Reference Numerals]

- 1: adhesive face
- 2: microneedle array
- 3: support film

## Claims

1. A repeelable support film for a microneedle patch, having a pattern-coated adhesive face on one side, and coming in contact with a back face of a substrate of a microneedle array through the adhesive face.

2. A repeelable support film for a water-soluble cosmetic sheet, having a pattern-coated adhesive face on one side, and coming in contact with a back face of the cosmetic sheet containing a water-soluble polymer through the adhesive face.

3. The support film according to claim 1, wherein an area of the pattern-coated adhesive face of the support film accounts for 0.1% to 30% of an area of the microneedle array.

4. The support film according to claim 2, wherein an area of the pattern-coated adhesive face of the support film accounts for 0.1% to 30% of an area of the water-soluble cosmetic sheet.

5. The support film according to any one of claims 1 to 4, wherein the pattern-coated adhesive face of the support film has a probe tack value of 0.1 N to 3.0 N.

6. A microneedle patch composed of a microneedle array containing a water-soluble polymer or a water-swellable resin as a base, and a support film, wherein the support film has a pattern-coated adhesive face on one side and adheres to a back face of a substrate of the microneedle array through the adhesive face.

7. A water-soluble sheet-shaped cosmetic composed of a water-soluble cosmetic sheet containing a water-soluble polymer and a support film, wherein the support film has a pattern-coated adhesive face on one side and adheres to a back face of the water-soluble cosmetic sheet through the adhesive face.

8. The microneedle patch according to claim 6, wherein an area of the pattern-coated adhesive face of the support film accounts for 0.1% to 30% of an area of the microneedle array.

9. The water-soluble sheet-shaped cosmetic according to claim 7, wherein an area of the pattern-coated adhesive face of the support film accounts for 0.1% to 30% of an area of the water-soluble cosmetic sheet.

10. The microneedle patch or the water-soluble sheet-shaped cosmetic according to any one of claims 6 to 9, wherein the pattern-coated adhesive face of the support film has a probe tack value of 0.1 N to 3.0 N.
